# EUROPEAN PATENT APPLICATION

(11) **EP 4 257 699 A1**
(43) Date of publication of application: **11.10.2023**
(21) Application number: 21903531.8
(22) Date of filing: 22.03.2021
(51) Int. Cl.: C12P 21/00, C12P 7/64, C12N 1/12, C12R 1/89

(54) **METHOD FOR PRODUCING BIOMASS COMPRISING PROTEIN AND OMEGA-3 FATTY ACIDS FROM SINGLE MICROALGAE, AND BIOMASS PRODUCED THEREBY**

(30) Priority: 07.12.2020 KR 20200169849
(71) Applicant: CJ Cheiljedang Corporation, Seoul 04560 (KR)
(72) Inventor: SHIN, Won Sub, Seoul 04560 (KR); JANG, Sung Hoon, Seoul 04560 (KR); KIM, Ji Young, Seoul 04560 (KR); CHOI, Jung Woon, Seoul 04560 (KR); KANG, Hae Won, Seoul 04560 (KR); GWAK, Jun Seok, Seoul 04560 (KR)
(74) Representative: Bates, Philip Ian
(86) International application number: PCT/KR2021/003477
(87) International publication number: WO 2022/124482

(57) **Abstract**

The present application relates to a method for producing biomass comprising protein and omega-3 fatty acids from single microalgae, and biomass produced thereby, the method for producing biomass according to one embodiment supplying the cultivation stage continuously with a nitrogen source to allow producing single microalgae-derived biomass having high protein and omega-3 fatty acid content, and as such, biomass produced thereby can be effectively used as the single microorganism source of protein and omega-3 fatty acids.

## Description

### [TECHNICAL FIELD]

The present application relates to a method for producing a biomass comprising protein and omega-3 fatty acids from single microalgae, and a biomass produced thereby.

### [BACKGROUND ART]

Unsaturated fatty acids are fatty acids having one or more double bonds in the fatty acid chain, and when fatty acids include two or more double bonds, they are called polyunsaturated fatty acids (PUFAs). Among them, docosahexaenoic acid (DHA) and eicosapentaenoic acid (EPA) are representative omega-3 fatty acids, which are fatty acids essential for brain, eye tissues, and nervous systems. In addition, they are known to play an important role in the development of the nervous system, such as visual acuity and motor neuron ability of infants, and the prevention of cardiovascular disease, and are the most abundant components in the structural lipids of the brain.

Industrially, the main source of supplying polyunsaturated fatty acids such as omega-3 fatty acids is fish oil extracted from oil of blue-backed fish, and fish meal is also widely used as a source of proteins in foods or feeds. However, due to the difficulty in continuously supplying fish oil and fish meal, such as limited catch, etc., there is a need to develop an alternative source of omega-3 fatty acids and proteins that may replace fish oil and fish meal.

Recently, studies on the production of polyunsaturated fatty acids by microalgae culture are being conducted. However, since the studies are focused on research aimed at producing oils such as fatty acids, studies on a method capable of producing a high content of proteins from microalgae are insufficient. Accordingly, the present inventors completed the present disclosure by deriving a method of preparing a biomass comprising omega-3 fatty acids and a high content of proteins from single microalgae.

### [Prior Art Documents]

### [Patent Documents]

(Patent Document 1) US Patent Publication NO. US 2016/0208297 A1

### [DISCLOSURE]

### [TECHNICAL PROBLEM]

An object of the present disclosure is to provide a method of preparing a biomass derived from *Thraustochytrid* microalgae, the method comprising culturing, in a medium, a single strain of the *Thraustochytrid* microalgae; and continuously supplying a nitrogen source during the culturing, and a biomass derived from single *Thraustochytrid* microalgae, which is prepared by the method.

### [TECHNICAL SOLUTION]

Each description and embodiment disclosed in the present application can also be applied to each other description and embodiment. In other words, all combinations of various elements disclosed in the present application fall within the scope of the present application. In addition, the scope of the present application is not be construed as being limited by specific description described below. In addition, those skilled in the art will recognize or confirm many equivalents to specific aspects of the present application described in the present application using only an ordinary experiment. Moreover, such equivalents are intended to be comprised in the present application.

In one aspect, the present application provides a method for preparing a biomass derived from *Thraustochytrid* microalgae, the method comprising culturing, in a medium, a single strain of the *Thraustochytrid* microalgae; and continuously supplying a nitrogen source during the culturing.

As used herein, the term "*Thraustochytrid*" refers to microalgae belonging to the order *Thraustochytriales.* The *Thraustochytrid* microalgae may be microalgae belonging to *Thraustochytrium* sp., *Schizochytrium* sp., *Aurantiochytrium* sp., or *Thraustochytriidae* sp.

As used herein, the term *"Thraustochytrium* sp.", *"Schizochytrium* sp.", "*Aurantiochytrium* sp.", or "*Thraustochytriidae* sp." is one of the genus belonging to the order *Thraustochytriales,* the family *Thraustochytriaceae,* and may be used interchangeably with the term "genus *Thraustochytrium",* "genus *Schizochytrium",* "genus *Aurantiochytrium",* or "genus *Thraustochytriidae*", respectively.

Further, the term "microalgae" refers to living organisms that are visible only through a microscope because they are not visible to the naked eye, among plants that photosynthesize with chlorophyll, and live floating in the water, and are also called phytoplankton.

In one specific embodiment, the *Thraustochytrid* microalgae may be, for example, microalgae of the *Thraustochytriidae* sp. CD01-6003, which was deposited with the Accession No. KCTC14346BP, or microalgae of the *Schizochytrium* sp. CD01-5004, which was deposited with the Accession No. KCTC14345BP, but is not limited thereto.

As used herein, the term "biomass" refers to organisms, such as plants, animals, microorganisms, etc., which may be used as chemical energy, that is, energy sources of bio-energy. The biomass ecologically also refers to the weight or energy amount of a specific living organism exiting at unit time and space. Further, although the biomass comprises compounds secreted by cells, it may also comprise, but is not limited to, cells and/or intracellular contents as well as extracellular materials. In the present disclosure, the biomass may be the *Thraustochytrid* microalgae itself, a cultured product thereof, a dried product thereof, a pulverized product thereof, a product produced by culturing or fermenting the microalgae, or may be a condensate of the biomass or a dried product of the biomass, but the biomass is not limited thereto.

The "cultured product" of the *Thraustochytrid* microalgae refers to a product obtained by culturing the microalgae, and specifically, may be a culture medium comprising the microalgae or a culture medium from which the microalgae is removed, but is not limited thereto. The "dried product" of the cultured product of the *Thraustochytrid* microalgae refers to a product obtained by removing moisture from the cultured product of the microalgae, for example, in the form of a dry cell body of the microalgae, but is not limited thereto. Further, the "pulverized product" of the dried product collectively refers to a product obtained by pulverizing the dried product which is obtained by removing moisture from the cultured product of the microalgae, for example, in the form of a dry cell body powder, but is not limited thereto.

The cultured product of the *Thraustochytrid* microalgae may be prepared according to a culturing method comprising inoculating the microalgae in a microalgae culture medium, and continuously supplying a nitrogen source during the culturing. The dried product of the cultured product and the pulverized product thereof may be prepared according to a method of treating or drying microalgae or a culture medium, which is known in the art.

With regard to the method of preparing the biomass, the nitrogen source may be supplied from immediately after inoculating the microalgae into a medium to the end of the culture.

Further, the nitrogen source may be continuously supplied such that the total nitrogen concentration in the culture medium is 300 ppm or more. For example, the nitrogen source may be continuously supplied such that the total nitrogen concentration in the culture medium is maintained within the range of 300 ppm to 10,000 ppm, 300 ppm to 8,000 ppm, 300 ppm to 5,000 ppm, 300 ppm to 3,000 ppm, 300 ppm to 2,000 ppm, 300 ppm to 1,500 ppm, 350 ppm to 10,000 ppm, 350 ppm to 8,000 ppm, 350 ppm to 5,000 ppm, 350 ppm to 3,000 ppm, 350 ppm to 2,000 ppm, or 350 ppm to 1,500 ppm.

With regard to the method of preparing the biomass, the medium may comprise a carbon source and a nitrogen source.

With regard to the method of preparing the biomass, the nitrogen source in continuously supplying the nitrogen source and the nitrogen source comprised in the medium may be i) any one or more organic nitrogen sources selected from the group consisting of a yeast extract, a beef extract, peptone, and tryptone, or ii) any one or more inorganic nitrogen sources selected from the group consisting of ammonium acetate, ammonium nitrate, ammonium chloride, ammonium sulfate, sodium nitrate, urea, monosodium glutamate (MSG), and ammonia, but the nitrogen source is not limited thereto, as long as it is used in culturing the *Thraustochytrid* microalgae.

The continuous supplying of the nitrogen source may be performed by, for example, continuously supplying a medium comprising the nitrogen source to the culture medium, or continuously supplying ammonia gas to a fermentor, where the microalgae is cultured, but is not limited thereto. The continuous supplying of the medium comprising the nitrogen source to the culture medium may be performed by, for example, a fed-batch culture method, in which the medium is intermittently supplied, or a continuous culture method, in which the medium is continuously supplied.

The method may further comprise continuously supplying a carbon source during the culturing. The continuous supplying of the carbon source may be performed by, for example, continuously supplying a medium comprising the carbon source to the culture medium, but is not limited thereto. The continuous supplying of the medium comprising the carbon source to the culture medium may be performed by, for example, a fed-batch culture method, or a continuous culture method. The continuous supplying of the carbon source may be performed while maintaining the carbon source concentration at 5% (w/v) or less in the culture medium.

The carbon source in continuously supplying the carbon source and the carbon source comprised in the medium may be any one or more selected from the group consisting of glucose, fructose, maltose, galactose, mannose, sucrose, arabinose, xylose, and glycerol, but the carbon source is not limited thereto, as long as it is used in culturing the *Thraustochytrid* microalgae.

The medium may further comprise appropriate phosphorus sources, inorganic compounds, amino acids, and/or vitamins, etc., which are used to culture the *Thraustochytrid* microalgae. For example, the phosphorus sources may include potassium dihydrogen phosphate, dipotassium hydrogen phosphate, sodium-containing salts corresponding thereto, etc., individually or in a mixture, but are not limited thereto.

The culturing may be performed for 95 hours or less. For example, the culturing may be performed for 30 hours to 95 hours, 35 hours to 95 hours, 40 hours to 95 hours, 45 hours to 95 hours, 30 hours to 90 hours, 35 hours to 90 hours, 40 hours to 90 hours, or 45 hours to 90 hours.

The culturing may be performed at 20 °C to 35 °C. For example, the culturing may be performed at 25 °C to 35 °C, 20 °C to 30 °C, or 25 °C to 30 °C, but is not limited thereto.

Further, during the culturing, to maintain the aerobic state of the cultured product, oxygen or oxygen-containing gas may be injected into the cultured product, or to maintain the anaerobic or non-aerobic state, no gas may be injected, or nitrogen, hydrogen, or carbon dioxide gas may be injected, but the present disclosure is not limited thereto.

The culturing may be performed while adjusting pH, for example, performed adjusting pH to maintain pH of 3.5 to 9.0, pH of 4.0 to 9.0, pH of 4.5 to 9.0, pH of 5.0 to 9.0, pH of 3.5 to 8.0, pH of 4.0 to 8.0, pH of 4.5 to 8.0, or pH of 5.0 to 8.0 using a basic compound (e.g., sodium hydroxide, potassium hydroxide, or ammonia) or an acidic compound (e.g., phosphoric acid or sulfuric acid), but is not limited thereto.

The method of preparing the biomass may further include recovering the biomass from the strain, the cultured product of the strain, the dried product of the cultured product, or the pulverized product of the dried product.

The recovering of the biomass may be collecting the desired biomass using an appropriate method known in the art. For example, centrifugation, filtration, anion exchange chromatography, crystallization, HPLC, etc. may be used. A purification process may be further included.

The biomass prepared by the above preparation method may comprise 50 wt% or more of proteins and 37 wt% or less of fats, based on the total weight of the biomass. The biomass may comprise, for example, 50 wt% to 80 wt%, 50 wt% to 75 wt%, 50 wt% to 70 wt%, 55 wt% to 80 wt%, 55 wt% to 75 wt%, or 55 wt% to 70 wt% of proteins; and 5 wt% to 37 wt%, 5 wt% to 35 wt%, 5 wt% to 30 wt%, 5 wt% to 25 wt%, 10 wt% to 37 wt%, 10 wt% to 35 wt%, 10 wt% to 30 wt%, 10 wt% to 25 wt%, 15 wt% to 37 wt%, 15 wt% to 35 wt%, 15 wt% to 30 wt%, or 15 wt% to 25 wt% of fats.

The biomass prepared by the above preparation method may comprise 50 wt% or more of proteins and omega-3 fatty acids, based on the total weight of the biomass. The omega-3 fatty acids may comprise any one or more of docosahexaenoic acid (DHA) and eicosapentaenoic acid (EPA).

As used herein, the term "DHA" is one of polyunsaturated fatty acids, represented by Formula C₂₂H₃₂O₂, and belongs to omega-3 fatty acid, together with α-linolenic acid (ALA) and EPA, and its trivial name is cervonic acid, and it may also be abbreviated as 22:6 n-3.

As used herein, the term "EPA" is one of polyunsaturated fatty acids, represented by Formula C₂₀H₃₀O₂, and belongs to omega-3 fatty acid, together with ALA and DHA, and may also be abbreviated as 20:5 n-3.

With regard to the biomass comprising 50 wt% or more of proteins and omega-3 fatty acids, the biomass may comprise 3 wt% to 30 wt%, 3 wt% to 25 wt%, 3 wt% to 20 wt%, or 3 wt% to 15 wt% of omega-3 fatty acids, based on the total weight of the biomass. Further, the biomass may comprise 15 wt% to 60 wt%, 15 wt% to 55 wt%, or 15 wt% to 50 wt% of omega-3 fatty acids, based on the weight of the total fatty acids.

Another aspect provides a biomass derived from single *Thraustochytrid* microalgae, which is prepared by a method of preparing the biomass, the method comprising culturing, in a medium, a single strain of the *Thraustochytrid* microalgae; and continuously supplying a nitrogen source during the culturing.

The method of preparing the biomass is the same as described above.

The biomass may comprise 50 wt% or more of proteins and 37 wt% or less of fats, based on the total weight of the biomass. The biomass may compris, for example, 50 wt% to 80 wt%, 50 wt% to 75 wt%, 50 wt% to 70 wt%, 55 wt% to 80 wt%, 55 wt% to 75 wt%, or 55 wt% to 70 wt% of proteins; and 5 wt% to 37 wt%, 5 wt% to 35 wt%, 5 wt% to 30 wt%, 5 wt% to 25 wt%, 10 wt% to 37 wt%, 10 wt% to 35 wt%, 10 wt% to 30 wt%, 10 wt% to 25 wt%, 15 wt% to 37 wt%, 15 wt% to 35 wt%, 15 wt% to 30 wt%, or 15 wt% to 25 wt% of fats.

The biomass may comprise, for example, 50 wt% or more of proteins and omega-3 fatty acids, based on the total weight of the biomass, wherein the omega-3 fatty acids may comprise any one or more of DHA and EPA.

With regard to the biomass comprising 50 wt% or more of proteins and omega-3 fatty acids, the biomass may comprise 3 wt% to 30 wt%, 3 wt% to 25 wt%, 3 wt% to 20 wt%, or 3 wt% to 15 wt% of omega-3 fatty acids, based on the total weight of the biomass. Further, the biomass may comprise 15 wt% to 60 wt%, 15 wt% to 55 wt%, or 15 wt% to 50 wt% of omega-3 fatty acids, based on the weight of the total fatty acids.

Still another aspect provides a composition comprising the biomass derived from single *Thraustochytrid* microalgae, or a condensate or dried product of the biomass, which is prepared by a method of preparing the biomass, the method comprising culturing, in a medium, the single strain of the *Thraustochytrid* microalgae; and continuously supplying a nitrogen source during the culturing.

The method of preparing the biomass and the biomass derived from single *Thraustochytrid* microalgae are the same as described above.

The concentrate or dried product of the biomass may be prepared according to a method of treating, concentrating, or drying a microbial biomass, which is known in the art.

The composition may be in the form of a solution, a powder, or a suspension, but is not limited thereto. The composition may be, for example, a food composition, a feed composition, or a feed additive composition.

As used herein, the term "feed composition" refers to a feed fed to animals. The feed composition refers to a material that supplies organic or inorganic nutrients necessary for maintaining animal's life, or producing meat, milk, etc. The feed composition may further comprise nutrients necessary for maintaining animal's life, or producing meat, milk, etc. The feed composition may be prepared into various types of feeds known in the art, and specifically, may include a concentrated feed, a coarse feed, and/or a specialized feed.

As used herein, the term "feed additive" refers to a material that is added to a feed for the purpose of various effects, such as nutrient replenishment, prevention of weight loss, improvement in digestive utilization of cellulose in feed, improvement of milk quality, prevention of reproductive disorder, improvement of conception rate, and prevention of high-temperature stress in summer. The feed additive of the present disclosure corresponds to a supplementary feed under the feed management law, and may further include a mineral preparation, such as sodium hydrogen carbonate, bentonite, magnesium oxide, complex mineral, etc., a mineral preparation that is trace mineral, such as zinc, copper, cobalt, selenium, etc., a vitamin preparation, such as carotene, vitamin A, vitamin D, vitamin E, nicotinic acid, vitamin B complex, etc., a protected amino acid preparation, such as methionine, lysine, etc., a protected fatty acid preparation, such as fatty acid calcium salt, etc., a live bacteria preparation, such as probiotic bacteria (lactic acid bacteria), yeast cultures, or mold fermentation products, etc., a yeast preparation, etc.

As used herein, the term "food composition" includes all types of foods, such as functional foods, nutritional supplements, health foods, and food additives. The above food composition may be prepared in various forms according to common methods known in the art.

The composition of the present disclosure may further comprise crops, such as pulverized or shredded wheat, oats, barley, corn, and rice; vegetable protein feeds, for example, feeds mainly consisting of soybeans and sunflowers; animal protein feeds, such as blood meal, meat meal, bone meal, and fish meal; sugar and dairy products, for example, various dry ingredients composed of milk powders and whey powder, and may further comprise nutritional supplements, digestion- and absorption-enhancers, growth promoters, etc.

The composition of the present disclosure may be administered to animals alone or in combination with other feed additives in an edible carrier. Further, the composition may be topdressing, may be directly mixed with feeds, or may be easily administered to animals as oral dosage forms separately from feeds. When administered separately from feeds, the composition may be combined with pharmaceutically acceptable edible carriers and prepared into immediate-release formulations or sustained-release formulations, as well known in the art. The edible carriers may be solid or liquid, for example, corn starch, lactose, sucrose, soy flake, peanut oil, olive oil, sesame oil, and propylene glycol. When solid carriers are used, the composition may be in a form of tablet, capsule, powder, troche or lozenge, or may be a not-dispersed topdressing. When liquid carriers are used, the composition may have a form of soft gelatin capsules, syrup, suspension, emulsion, or solution.

The composition of the present disclosure may comprise, for example, preservatives, stabilizers, wetting agents, emulsifiers, cryoprotectants, excipients, etc. The cryoprotectants may be one or more selected from the group consisting of glycerol, trehalose, maltodextrin, skim milk powder, and starch.

The preservatives, stabilizers, or excipients may be comprised in the composition in an effective amount sufficient to reduce deterioration of the *Thraustochytrid* microalgae which is comprised in the composition. Further, the cryoprotectant may be comprised in the composition in an effective amount sufficient to reduce deterioration of the *Thraustochytrid* microalgae which is comprised in the composition, when the composition is in a dried state.

The composition may be added to animal feeds by means of dipping, spraying, or mixing for use.

The composition of the present disclosure may be applied to a diet for various animals such as mammals, birds, fish, crustaceans, cephalopods, reptiles, and amphibians. For example, the mammals may include pigs, cows, sheep, goats, laboratory rodents, pets, etc., the birds may include poultry, which may include chickens, turkeys, ducks, geese, pheasant, quail, etc., but are not limited thereto. Further, the fish may include commercial farmed fish and juvenile fish thereof, ornamental fish, etc., and the crustaceans may include shrimp, barnacles, etc., but are not limited thereto. The composition may also be applied to a feed for rotifer, which is an animal plankton.

### [ADVANTAGEOUS EFFECTS]

According to the method of preparing a biomass according to an aspect, it is possible to prepare a biomass derived from single microalgae, which comprises high contents of proteins and omega-3 fatty acids, by continuously supplying nitrogen sources during the culturing process. The biomass prepared thereby may be used as a single microbial source of proteins and omega-3 fatty acids.

### [MODE FOR INVENTION]

Hereinafter, the present disclosure will be described in more detail with reference to exemplary embodiments. However, these exemplary embodiments are only for illustrating one or more specific embodiments, and the scope of the present disclosure is limited by these exemplary embodiments.

### Example 1. Examination of Production Amounts of Fats and Proteins according to Method of Culturing Microalgae of Thraustochytriidae sp.

### 1-1. Preparation and Seed culture of Microalgae

The microalgae of the *Thraustochytriidae* sp. CD01-6003 (Accession No: KCTC14346BP) was seeded in a sterile MJW01 medium (30 g/L of glucose, 3.0 g/L of MgSO₄·7H₂O, 10 g/L of Na₂SO₄, 1.0 g/L of NaCl, 9.0 g/L of yeast extract, 1.0 g/L of MSG 1H₂O, 1.0 g/L of NaNO₃, 0.1 g/L of KH₂PO₄, 0.5 g/L of K₂HPO₄, 0.5 g/L of CaCl₂, and 10 mL/L of a vitamin mixed solution), and cultured in a 250 mL flask under conditions of 20 °C to 35 °C and 100 rpm to 300 rpm for 10 hours to 30 hours.

### 1-2. Main culture

### 1-2-1. Comparative Example (1)

The seed culture prepared in Example 1-1 was dispensed in a 5 L fermentor containing a sterile MJW01 medium supplemented with 6 g/L of ammonium sulfate ((NH₄)₂·SO₄) and 5 g/L of yeast extract as nitrogen sources, and cultured under conditions of 20 °C to 35 °C, 100 rpm to 500 rpm, and 0.5 vvm to 1.5 vvm for a total of 101 hours until the volume of the culture in the fermentor reached 2.8 L. Immediately after starting the culturing with additional nitrogen sources, ammonia gas was continuously supplied to the fermentor for 14 hours such that the total nitrogen concentration in the culture medium was 300 ppm or more, and then, the supply of ammonia gas was stopped. During the entire culturing process, a feed containing a carbon source was continuously supplied such that the concentration of the carbon source in the culture medium was maintained below 5%.

### 1-2-2. Example (1)

Culturing was performed in the same manner as in 1-2-1 for a total of 86 hours, except that, during the entire culturing process, ammonia gas was continuously supplied to the fermentor such that the total nitrogen concentration in the culture medium was 300 ppm or more.

### 1-2-3. Example (2)

Culturing was performed in the same manner as in 1-2-1 for a total of 75 hours, except that, during the entire culturing process, ammonia gas was continuously supplied to the fermentor such that the total nitrogen concentration in the culture medium was 300 ppm or more, and only 18 g/L of ammonium sulfate as the nitrogen source was added to the sterile MJW01 medium.

### 1-2-4. Example (3)

Culturing was performed in the same manner as in 1-2-1 for a total of 55 hours, except that, during the entire culturing process, ammonia gas was continuously supplied to the fermentor such that the total nitrogen concentration in the culture medium was 300 ppm or more, and 10 g/L of ammonium sulfate as the nitrogen source was further added to the feed containing the carbon source.

### 1-2-5. Example (4)

Culturing was performed in the same manner as in 1-2-1 for a total of 92 hours, except that, during the entire culturing process, ammonia gas was continuously supplied to the fermentor such that the total nitrogen concentration in the culture medium was 300 ppm or more, only 6 g/L of ammonium sulfate as the nitrogen source was added to the sterile MJW01 medium, and 12 g/L of ammonium sulfate as the nitrogen source was further added to the feed containing the carbon source.

### 1-3. Analysis of fat content

Each of the microalgae cultures cultured in 1-2-1 to 1-2-5 was collected and centrifuged. Then, the obtained cells were washed with PBS three times, and dried at 60 °C for 16 hours. A hydrolysis reaction was allowed at 80 °C by adding an 8.3 M hydrochloric acid solution to 2 g of dried cells. Thereafter, 30 mL of ethyl ether and 20 mL of petroleum ether were added to the reaction product, and mixing for 30 seconds and centrifugation were repeated three times or more. A separated solvent layer was transferred to a pre-weighed round flask, and then dried in a container, from which the solvent and residual moisture were removed, through nitrogen (N₂) purging. The total oil content was calculated by measuring the weight of oil which remained after drying the solvent. The content of omega-3 fatty acids (DHA and EPA) in the oil was measured by gas chromatography after being pretreated with 0.5 N methanolic NaOH and 14% trifluoroborane methanol (BF₃-MeOH).

**[Table 1]**

| Conditions | Total fatty acid/Biomass( %) | EPA/Tot al fatty acid(%) | DHA/Tot al fatty acid(%) | Omeg a-3/Total fatty acid( %) | EPA/Biomass( %) | DHA/Biomass( %) | Omega-3/biomass( %) |
|---|---|---|---|---|---|---|---|
| Comparati ve Example(1 ) | 43.42 | 0.49 | 10.06 | 10.55 | 0.21 | 4.37 | 4.58 |
| Example(1 ) | 28.90 | 2.96 | 24.39 | 27.35 | 0.86 | 7.05 | 7.90 |
| Example(2 ) | 14.97 | 2.54 | 26.58 | 29.12 | 0.38 | 3.98 | 4.36 |
| Example(3 ) | 26.13 | 0.86 | 16.75 | 17.61 | 0.22 | 4.38 | 4.60 |
| Example(4 ) | 17.91 | 2.71 | 36.12 | 38.83 | 0.49 | 6.47 | 6.95 |

As a result, as shown in Table 1, the total fatty acid (TFA) content in the biomass was more reduced in Examples (1) to (4), in which culturing was performed while continuously supplying the nitrogen sources, as compared with Comparative Example(1). However, the EPA, DHA, and omega-3 contents in the total fatty acids in Examples (1) to (4) were equivalent to or higher than those of Comparative Example (1).

### 1-4. Analysis of protein content

With respect to each 0.5 g to 1 g of the dried cells obtained in the same manner as in 1-3, the nitrogen content in the sample was quantitatively analyzed using an elemental analyzer. The weight ratio (TN%) of nitrogen present in each sample was multiplied by 6.25 and calculated as the total protein content in the sample.

**[Table 2]**

| Conditions | Total protein/Biomass(%) |
|---|---|
| Comparative Example(1) | Up to about 43.58 ^{∗} |
| Example(1) | Up to about 58.10 ^{∗} |
| Example(2) | 55.7 |
| Example(3) | 58.0 |
| Example(4) | 57.5 |

| | |
|---|---|
| (*: estimate) | |

As a result, as shown in Table 2, all of Examples (2) to (4) showed the total protein content of 55% or more, and Example (1) showed the total fatty acid content of 28.9%, as measured in 1-3. Considering that the microalgae biomass generally has the carbohydrate content of about 2% to about 3%, and the ash content of about 8% to about 10%, it may be estimated that the total protein content may reach up to about 58.10%. In addition, Comparative Example (1) showed the total fatty acid content of 43.42%, as measured in 1-3, estimating that the maximum total protein content is merely about 43.58%. These results confirmed that when the microalgae are cultured under the condition of continuously supplying the nitrogen source, proteins may be produced with a high content of 55% or more.

### Example 2. Examination of Production Amounts of Fats and Proteins according to Method of Culturing Microalgae of Schizochytrium sp.

### 2-1. Preparation and Seed culture of Microalgae

Seed culture of the microalgae of the *Schizochytrium* sp. CD01-5004 (Accession No: KCTC14345BP) was performed in the same manner as in Example 1-1.

### 2-2. Main culture

### 2-2-1. Comparative Example (2)

The seed culture prepared in Example 2-1 was dispensed in a 5 L fermentor containing a sterile MJW01 medium, and cultured under conditions of 20 °C to 35 °C, 100 rpm to 500 rpm, and 0.5 vvm to 1.5 vvm for a total of 105 hours until the volume of the culture in the fermentor reached 2.8 L. Immediately after starting the culturing with additional nitrogen sources, ammonia gas was continuously supplied to the fermentor for 10 hours such that the total nitrogen concentration in the culture medium was 300 ppm or more, and then, the supply of ammonia gas was stopped. During the entire culturing process, a feed containing a carbon source was continuously supplied such that the concentration of the carbon source in the culture medium was maintained below 5%.

### 2-2-2. Example (5)

Culturing was performed in the same manner as in 2-2-1 for a total of 84 hours, except that, during the entire culturing process, ammonia gas was continuously supplied to the fermentor such that the total nitrogen concentration in the culture medium was 300 ppm or more, and 6 g/L of ammonium sulfate and 10 g/L of yeast extract as the nitrogen sources were further added to the sterile MJW01 medium.

### 2-2-3. Example (6)

Culturing was performed in the same manner as in 2-2-2, except that culturing was performed for a total of 53 hours.

### 2-2-4. Examples (7) and (8)

Culturing was performed in the same manner as in 2-2-2, except that culturing was performed for a total of 50 hours.

### 2-2-5. Example (9)

Culturing was performed in the same manner as in 2-2-2, except that culturing was performed for a total of 47 hours.

### 2-2-6. Example (10)

Culturing was performed in the same manner as in 2-2-2 for a total of 47 hours, except that 5 g/L of MSG as the nitrogen source was further added to the sterile MJW01 medium.

### 2-3. Analysis of fat content

Each of the microalgae cultures cultured in 2-2-1 to 2-2-6 was collected, and the fat contents thereof were analyzed in the same manner as in Example 1-3.

**[Table 3]**

| Conditions | Total fatty acid/Biomass( %) | EPA/Tot al fatty acid(%) | DHA/Tot al fatty acid(%) | Omeg a-3/Tota 1 fatty acid( %) | EPA/Biomass( %) | DHA/Biomass( %) | Omega-3/Biomass( %) |
|---|---|---|---|---|---|---|---|
| Comparati ve Example(2 ) | 41.91 | 2.53 | 39.18 | 41.71 | 1.06 | 16.42 | 17.48 |
| Example(5 ) | 24.39 | 4.25 | 42.92 | 47.17 | 1.04 | 10.47 | 11.50 |
| Example(6 ) | 20.12 | 3.90 | 37.81 | 41.70 | 0.78 | 7.60 | 8.39 |
| Example(7 ) | 20.95 | 3.68 | 39.54 | 43.22 | 0.77 | 8.28 | 9.05 |
| Example(8 ) | 19.99 | 4.50 | 38.84 | 43.34 | 0.90 | 7.76 | 8.66 |
| Example(9 ) | 24.90 | 3.98 | 39.27 | 43.25 | 0.99 | 9.78 | 10.77 |
| Example(1 0) | 25.04 | 5.07 | 42.02 | 47.09 | 1.27 | 10.52 | 11.79 |

As a result, as shown in Table 3, the total fatty acid (TFA) content in the biomass was more reduced in Examples (5) to (10), in which culturing was performed while continuously supplying the nitrogen sources, as compared with Comparative Example (2). However, the EPA, DHA, and omega-3 contents in the total fatty acids in Examples (5) to (10) were equivalent to or higher than those of Comparative Example (2).

### 2-4. Analysis of protein content

Each of the microalgae cultures cultured in 2-2-1 to 2-2-6 was collected, and the protein contents thereof were analyzed in the same manner as in Example 1-4.

**[Table 4]**

| Conditions | Total protein/Biomass(%) |
|---|---|
| Comparative Example(2) | Up to about 45.1^{∗} |
| Example(5) | 62.4 |
| Example(6) | 64.9 |
| Example(7) | 65.6 |
| Example(8) | 66.3 |
| Example(9) | 60.7 |
| Example(10) | 58.7 |

| | |
|---|---|
| (*: estimate) | |

As a result, as shown in Table 4, all of Examples (5) to (10) showed the total protein content of 58% or more, and Comparative Example (2) showed the total fatty acid content of 41.91%, as measured in 2-3. Considering that the microalgae biomass generally has the carbohydrate content of about 2% to about 3%, and the ash content of about 8% to about 10%, it may be estimated that the total protein content may reach up to about 45.1%. These results confirmed that when the microalgae are cultured under the condition of continuously supplying the nitrogen source, proteins may be produced with a high content of 58% or more.

### 2-5. Analysis of amino acid content and composition

The microalgae culture of Example (10) cultured in 2-2-6 was collected, and the dry cells were obtained in the same manner as in 1-3, and 0.5 g to 1 g of the dry cells were subjected to acid hydrolysis. Thereafter, the resultant was subjected to liquid chromatography, and the total amino acid content and the contents of individual amino acids were analyzed. The concentrations of individual amino acids in the sample were normalized to the amount of dry cells used to calculate the contents (%) of individual amino acids relative to the weight of the dry cells, and the total amino acid content (%) relative to the weight of the dry cells was calculated by summing the contents of all detected amino acids.

**[Table 5]**

| | Content in biomass (%) | | Content in biomass (%) | | Content in biomass (%) |
|---|---|---|---|---|---|
| Total amino acids | 43.09 | Alanine | 2.50 | Tyrosine | 1.24 |
| Aspartic acid | 3.62 | Cysteine | 0.56 | Phenylalanine | 1.63 |
| Threonine | 1.80 | Valine | 2.06 | Lysine | 2.27 |
| Serine | 2.02 | Methionine | 0.82 | Histidine | 0.78 |
| Glutamate | 12.47 | Isoleucine | 1.36 | Arginine | 6.11 |
| Glycine | 0.00 | Leucine | 2.77 | Proline | 1.07 |

**[Table 6]**

| | Content in total amino acids (%) | | Content in total amino acids (%) | | Content in total amino acids (%) |
|---|---|---|---|---|---|
| Total amino acids | 100.00 | Alanine | 5.81 | Tyrosine | 2.88 |
| Aspartic acid | 8.39 | Cysteine | 1.30 | Phenylalanine | 3.79 |
| Threonine | 4.18 | Valine | 4.78 | Lysine | 5.27 |
| Serine | 4.69 | Methionine | 1.90 | Histidine | 1.82 |
| Glutamate | 28.93 | Isoleucine | 3.15 | Arginine | 14.19 |
| Glycine | 0.00 | Leucine | 6.43 | Proline | 2.48 |

As a result, as shown in Tables 5 and 6, Example (10), in which the microalgae were cultured under conditions of continuously supplying the nitrogen sources, showed the total protein content of 43.09% in the biomass, and had the highest content of glutamate among the individual amino acids, and its content ratio of glutamate to arginine was 2.04.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A method of preparing a biomass derived from *Thraustochytrid* microalgae, wherein the method comprising:
culturing, a single strain of the *Thraustochytrid* microalgae in a medium; and
continuously supplying a nitrogen source during the culturing.

2. The method according to claim 1, wherein the nitrogen source is supplied from immediately after inoculating the microalgae into the medium to the end of the culture.

3. The method according to claim 1, wherein the nitrogen source is continuously supplied such that the total nitrogen concentration in the culture medium is 300 ppm or more.

4. The method according to claim 1, wherein the medium comprises a carbon source and a nitrogen source.

5. The method according to claim 1 or 4, wherein the nitrogen source is i) any one or more organic nitrogen sources selected from the group consisting of a yeast extract, a beef extract, peptone, and tryptone, or ii) any one or more inorganic nitrogen sources selected from the group consisting of ammonium acetate, ammonium nitrate, ammonium chloride, ammonium sulfate, sodium nitrate, urea, monosodium glutamate (MSG), and ammonia.

6. The method according to claim 4, wherein the carbon source is any one or more selected from the group consisting of glucose, fructose, maltose, galactose, mannose, sucrose, arabinose, xylose, and glycerol.

7. The method according to claim 1, wherein the *Thraustochytrid* microalgae is microalgae of *Thraustochytrium* sp., *Schizochytrium* sp., *Aurantiochytrium* sp., or *Thraustochytriidae* sp.

8. The method according to claim 1, further comprising recovering the biomass from the strain, a cultured product of the strain, a dried product of the cultured product, or a pulverized product of the dried product.

9. The method according to claim 1, wherein the biomass comprises 50 wt% or more of proteins and 37 wt% or less of fats, based on the total weight of the biomass.

10. The method according to claim 1, wherein the biomass comprises 50 wt% or more of proteins, and omega-3 fatty acids, based on the total weight of the biomass.

11. The method according to claim 10, wherein the omega-3 fatty acid comprises any one or more of docosahexaenoic acid (DHA) and eicosapentaenoic acid (EPA).

12. The method according to claim 10, wherein the biomass comprises 15 wt% to 60 wt% of omega-3 fatty acids, based on the weight of the total fatty acids.

13. A biomass derived from single *Thraustochytrid* microalgae, which is prepared by a method of preparing the biomass, wherein the method comprising:
culturing a single strain of the *Thraustochytrid* microalgae in a medium; and
continuously supplying a nitrogen source during the culturing.

14. The biomass according to claim 13, wherein the biomass comprises 50 wt% or more of proteins and 37 wt% or less of fats, based on the total weight of the biomass.

15. The biomass according to claim 13, wherein the biomass comprises 50 wt% or more of proteins and omega-3 fatty acids, based on the total weight of the biomass.

16. The biomass according to claim 15, wherein the omega-3 fatty acid comprises any one or more of docosahexaenoic acid and eicosapentaenoic acid.

17. The biomass according to claim 15, wherein the biomass comprises 15 wt% to 60 wt% of omega-3 fatty acids, based on the weight of the total fatty acids.
